(19) Europäisches Patentamt
European Patent Office
Office européen des brevets

(11) Veröffentlichungsnummer: **0 194 529 B1**

(12) **EUROPÄISCHE PATENTSCHRIFT**

(45) Veröffentlichungstag der Patentschrift: **02.09.92**

(51) Int. Cl.$^5$: **A61K 31/415**

(21) Anmeldenummer: **86102641.7**

(22) Anmeldetag: **28.02.86**

(54) **Verwendung von 2-(Aminophenyl)-benzimidazol-Derivaten für die Herstellung von Arzneimitteln zur Bekämpfung maligner und nicht maligner proliferativer sowie autoimmuner Erkrankungen und zur Immunsuppression bei Transplantationen.**

(30) Priorität: **01.03.85 DE 3507222**
**28.06.85 DE 3523121**

(43) Veröffentlichungstag der Anmeldung:
**17.09.86 Patentblatt 86/38**

(45) Bekanntmachung des Hinweises auf die Patenterteilung:
**02.09.92 Patentblatt 92/36**

(84) Benannte Vertragsstaaten:
**AT CH DE FR GB IT LI**

(56) Entgegenhaltungen:
**EP-A- 0 116 967**
**BE-A- 631 490**

**J. PHARM. PHARMAC., Band 21, 1969, Seiten 573-576 V.V. SUBBA REDDY et al.: "The effects of 1-(di(2-chloroethyl)-aminoethyl)benzimiazole and related compounds on the growth of experimental tumours"**

**"Reallexikon der Medizin", Urban & Schwarzenberg, "Immunosuppression"**

**Berenbaum M.C., Immunosuppressive Agents : The Design of Selective Therapeutic Schedules, Antibiotica et Chemotherapia, 15, 155-176 (Karger, 1969)**

(73) Patentinhaber: **GÖDECKE AKTIENGESELLSCHAFT**
**Salzufer 16**
**W-1000 Berlin 10(DE)**

(72) Erfinder: **Weiershausen, Ute**
**Bundesstrasse 70**
**W-7803 Gundelfingen(DE)**
Erfinder: **Satzinger, Gerhard, Dr.**
**Im Mattenbühl 7**
**W-7809 Denzlingen(DE)**
Erfinder: **Herrmann, Wolfgang, Dr.**
**Zum Baumgarten 13**
**W-7802 Merzhausen(DE)**

**EP 0 194 529 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der DE-OS 33 05 755 sind N-Phenyl-benzamid-Derivate der allgemeinen Formel I bekannt.

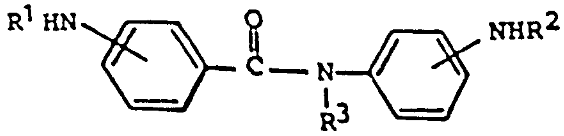

I

in welcher die Reste $R^1$, $R^2$ und $R^3$, die gleich oder verschieden sein können, ein Wasserstoffatom oder eine Methylgruppe bedeuten.

Sie dienen der Bekämpfung von malignen, proliferativen und autoimmunen Erkrankungen sowie der immunosuppressiven Therapie bei Transplantationen.

Die Verbindungen der Formel I passieren aufgrund ihrer Lipophilie überraschend die Bluthirnschranke und beeinflussen daher insbesondere Tumoren des ZNS-Systems.

Es wurde nun gefunden, daß die obengenannten Verbindungen der allgemeinen Formel I, dann wenn die Gruppe -$NHR^2$ in ortho-Stellung steht und der Rest $R^2$ ein Wasserstoffatom bedeutet, im sauren Milieu einer Ringkondensation unterliegen und Verbindungen der allgemeinen Formel II

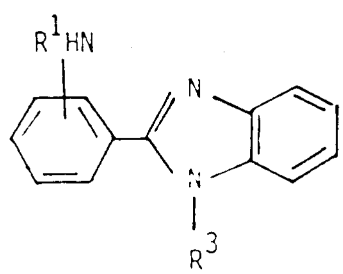

II

in welcher $R^1$ und $R^3$ die obengenannte Bedeutung haben bilden.

Nach dem bekannten Stand der Technik ist von Verbindungen dieser Struktur lediglich eine anthelmintische Wirkung beschrieben worden, (Vgl. BE-PS 631 490) die sie zusammen mit Phenothiazin aufweisen. Weiterhin ist von Benzimidazol-Derivaten eine antivirale Wirkung bekannt [C.A. 64, 1966, 2434 f].

Es wurde nun überraschend gefunden, daß die Verbindungen der allgemeinen Formel II sich zur Bekämpfung maligner und nicht maligner proliferativer sowie autoimmuner Erkrankungen und zur Immunsuppression bei Transplantationen eignen. Maligne proliferative Erkrankungen sind in erster Linie maligne Tumoren.

Die Verbindungen II können unter anderem dadurch hergestellt werden, daß man sie in einem geeigneten Lösungsmittel löst und im pH Bereich unterhalb 5, vorzugsweise unterhalb 3 der Ringkondensation unterwirft.

Unter die Verbindungen der allgemeinen Formel II fallen insbesondere solche Einzelverbindungen, bei denen die Gruppe $R^1$NH- in para-Stellung zum Benzimidazolringsystem steht. Hierzu gehören insbesondere:

2-(4-Aminophenyl)-benzimidazol,
2-(4-Methylaminophenyl)-benzimidazol,
2-(4-Aminophenyl)-(1-methyl)-benzimidazol,
2-(4-Methylaminophenyl)-(1-methyl)-benzimidazol.

Da die Verbindungen II basisch sind, bilden sie mit Säuren stabile Salze.

Als solche Salze kommen alle therapeutisch verwendbaren Säureadditionssalze in Betracht, wie z.B. Salze mit Halogenwasserstoffsäuren, wie Chlor- oder Bromwasserstoffsäure, Schwefelsäuren. Phosphorsäuren, Salpetersäure, aliphatischen, alicyclischen. aromatischen oder heterocyclischen Carbon- oder Sulfonsäuren, wie Ameisen-, Essig-, Propion-, Bernstein-, Glykol-, Milch-, Äpfel-, Wein-, Zitronen-, Ascorbin-, Malein-, Hydroxymalein- oder Brenztraubensäure; Phenylessig-, Benzoe-, p-Aminobezoe-, Anthranil-, p-Hydroxybenzoe-, Salicyl- oder p-Aminosalicylsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Methionin, Tryptophan, Lysin oder Arginin.

Die erfindungsgemäßen Wirkstoffe werden vorteilhaft in Form eines pharmazeutischen Präparats verabreicht, welches die Wirkstoffe in freier Form oder in Form einer ihrer therapeutisch verwendbaren Salze, in Mischung mit einem z.B. für die topische, enterale, z.B. orale oder rectale, oder vor allem parenterale, wie intramuskuläre oder intravenöse Applikation geeigneten pharmazeutischen organische oder anorganischen, festen oder flüssigen Trägermaterial enthält. Für die Bildung derselben kommen solche Stoffe in Frage, die mit den neuen Verbindungen nicht reagieren, wie z.B. Gelatine, Lactose, Stärke, Stearylalkohol, Magnesiumstearat, Talk, pflanzliche Öle, Benzylalkohole, Propylenglykole, Vaseline oder andere Arzneimittelträger.

Die pharmazeutischen Präparate können z.B. als Tabletten, Dragées, Kapseln, Suppositorien, Salben, Cremes oder in flüssiger Form als Lösungen, Suspensionen oder Emulsionen vorliegen. Gegebenenfalls sind sie sterilisiert und/oder enthalten Hilfsstoffe, wie Konservierungs-, Stabilisierungs-, Netz- oder Emulgiermittel, Lösungsvermittler oder Salze zur Veränderung des osmotischen Druckes oder Puffer. Sie können auch weitere Wirkstoffe enthalten.

Die anzuwendende Dosis hängt von der Art des zu therapierenden Krankheitsgeschehens und von individuellen Faktoren ab.

Im allgemeinen werden Dosen von 10 bis 300 mg, insbesondere 20 bis 100 mg verabreicht. In besonderen Fällen kann die Einzeldosis auch höher liegen.

Nachfolgend sind die wichtigsten auf Seite B2 aufgeführten Verbindungen näher erläutert:

2-(4-Aminophenyl)-benzimidazol

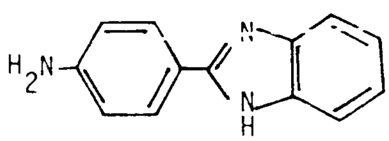

$C_{13}H_{11}N_3$
MG: 209,25

Die Verbindung wird hergestellt durch Kochen von 4-Aminobenzoyl-o-phenylen-diamin mit Salzsäure am Rückfluß. Schmp. 240 °C.

2-(4-Methylaminophenyl)-benzimidazol

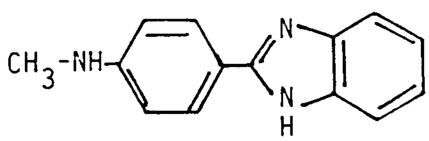

$C_{14}H_{13}N_3$
MG: 223,28

Die Verbindung entsteht beim Kochen von 4-(Acetyl-methylamino)-benzoyl-o-phenylendiamin mit Salzsäure in Ethanol. Schmp. 250 °C.

2-(4-Aminophenyl)-(1-methyl)-benzimidazol

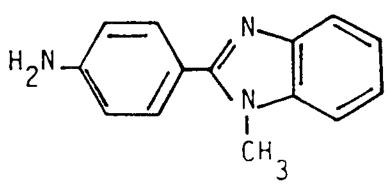

$C_{14}H_{13}N_3$
MG: 223,28

Die Verbindung entsteht beim Erhitzen von N-(4-Aminobenzoyl)-N-methyl-o-phenylendiamin in Methanol. Schmp. 181-2 °C.

**Patentansprüche**

1. Verwendung von Verbindungen der allgemeinen Formel II

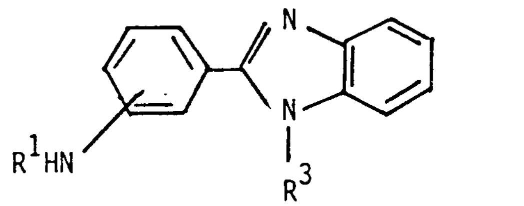

II

in welcher die Reste $R^1$ und $R^3$ ein Wasserstoffatom oder eine Methylgruppe bedeuten,

sowie von deren pharmakologisch verträglichen Salzen für die Herstellung von Arzneimitteln zur Bekämpfung maligner und nicht maligner proliferativer sowie autoimmuner Erkrankungen und zur Immunsuppression bei Transplantationen.

2. Verwendungen von Verbindungen gemäß Anspruch I, worin die Gruppe $R^1$HN- in para-Stellung zum Benzimidazol-Ringsystem steht.

**Claims**

1. Application of compounds of the general formula II

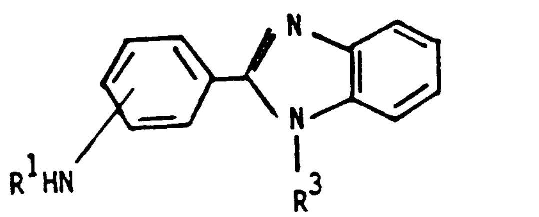

II

in which the radicals $R^1$ and $R^3$ signify a hydrogen atom or a methyl group,

as well as pharmacologically compatible salts thereof for the production of medicaments for combatting malign and non-malign proliferative as well as auto-immune diseases and for immunosuppression in transplantations.

2. Applications of compounds according to claim 1, in which the $R^1$HN- group is in the para-position with respect to the benzimidazole cyclic system.

**Revendications**

1. Utilisation de dérivés de formule générale II:

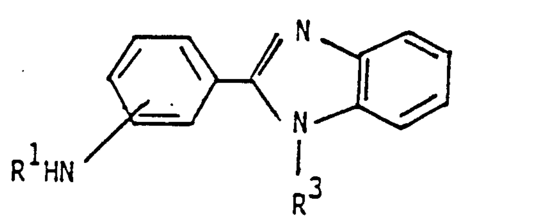

II

dans laquelle:

les restes $R^1$ et $R^3$ représentent un atome d'hydrogène ou un groupe méthyle,

ainsi que leurs sels pharmacologiquement acceptables pour la préparation de médicaments destinés au

traitement de maladies malignes et non-malignes prolifératives ainsi qu'autoimmunes et pour l'immunosuppression lors des transplantations.

**2.** Utilisations de dérivés selon la revendication 1, caractérisées en ce que le groupe $R^1HN$- se trouve en position para par rapport au système cyclique du benzimidazole.